(19) Europäisches Patentamt
European Patent Office
Office européen des brevets

(11) Publication number: 0 113 945
A1

(12) **EUROPEAN PATENT APPLICATION**

(21) Application number: 83300259.5

(22) Date of filing: 19.01.83

(51) Int. Cl.³: C 07 D 235/10, A 01 N 43/52

(43) Date of publication of application: 25.07.84
Bulletin 84/30

(84) Designated Contracting States: BE CH DE FR GB IT LI LU NL SE

(71) Applicant: ELI LILLY AND COMPANY, 307, East McCarty Street, Indianapolis Indiana 46285 (US)

(72) Inventor: O'Doherty, George Oliver Plunkett, Box 260 R.R. 1, Greenfield Indiana 46140 (US)

(74) Representative: Crowther, Terence Roger et al, Erl Wood Manor, Windlesham Surrey GU20 6PH (GB)

(54) **Amine salts.**

(57) Amine salts of benzimidazole compounds, of the formula

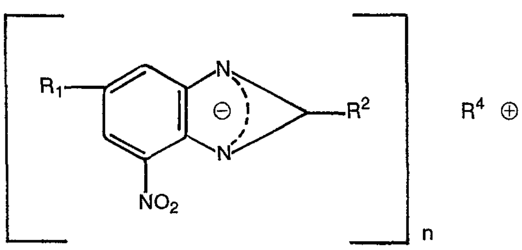

wherein $R^1$ is bromo, chloro, $-CF_3$, $-CF_2Br$, or $-CF_2Cl$; $R^2$ is perfluoroalkyl of $C_1$-$C_3$ or $-CF_2-CF_2R^3$ wherein $R^3$ is hydrogen or $C_1$-$C_6$ alkyl; $R^4$ is a substituted ammonium cation derived from an organic amine; and n is the valence of $R^4$, the said salt having a $K_{OW} > 150$ in the system octanol/water, are useful as ectoparasiticides and as agents for the control of colonial insects.

EP 0 113 945 A1

ACTORUM AG

# AMINE SALTS

The present invention concerns novel salts of certain benzimidazole compounds, which salts are useful for the control of insects. The novel salts are of the formula

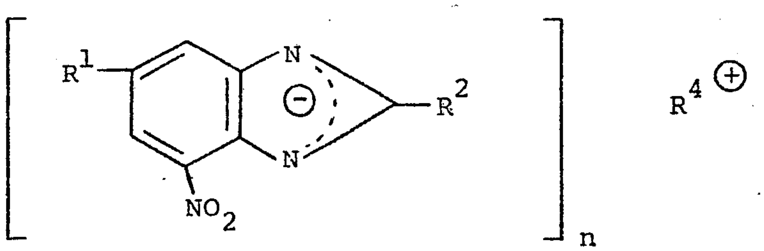

(I)

wherein $R^1$ is bromo, chloro, $-CF_3$, $-CF_2Br$, or $-CF_2Cl$; $R^2$ is perfluoroalkyl of $C_1$-$C_3$ alkyl or $-CF_2-CF_2R^3$ wherein $R^3$ is hydrogen or $C_1$-$C_6$ alkyl; $R^4$ is a substituted ammonium cation derived from an organic amine ; and n is the valence of $R^4$, the said salts having a $K_{OW}$ >150 in the system octanol/water.

The present salts are prepared by conventional procedures. The benzimidazole salts of formula I are prepared by reacting a compound of the formula

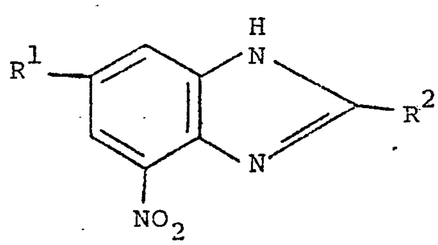

(II)

wherein $R^1$ and $R^2$ are defined as before

(1) with an organic amine, $R^4$, which is defined as before, in the presence of an aprotic solvent; or

(2) with an alkali metal hydroxide or an alkali metal carbonate, followed by reacting the alkali metal salt with an appropriate salt of the $R^4$ organic amine, in the presence of a protic solvent; or

(3) with an organic amine, $R^4$, which is defined as before, or with an appropriate salt of the $R^4$ organic amine, in the presence of a protic solvent;

and step (2) or (3) above is further followed by drying the resulting benzimidazoline salt of the formula

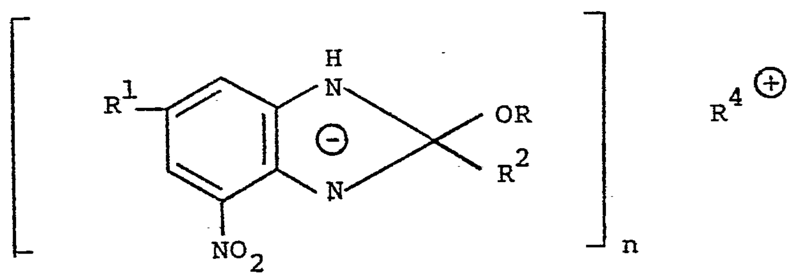

(III)

wherein $R^1$, $R^2$, $R^4$ and n are defined as before, and R is hydrogen or $C_1$-$C_4$ alkyl.

Also contemplated by the present invention is an ectoparasiticidal formulation comprising as active ingredient a benzimidazole salt of formula I,

associated with one or more acceptable carriers or diluents therefore. In this formulation the amount of active ingredient of formula I preferably present is from 1 to 98% by weight.

The substituted ammonium cation derived from an organic amine, $R^4$, is generated by protonation of a primary, secondary or tertiary organic amine or is a quarternary radical. The methods used to prepare this cation are conventional. The identity of the substituted ammonium cation is not critical so long as it confers substantial lipophilicity on the salt. The salt should have a partition coefficient in the system of n-octanol and water of $K_{OW}$ >150.

The alkali metal hydroxide or alkali metal carbonates that are used in preparing the salts of formula I are preferably, sodium or potassium hydroxide and sodium or potassium carbonate, and more preferably sodium hydroxide.

The reaction is carried out in a suitable solvent, preferably aprotic solvents such as the ethers, e.g. diethyl ether, and halogenated hydrocarbons, preferably methylene chloride. Protic solvents such as water and the $C_1$-$C_4$ alkanols can be employed; however, their use favors the conversion of the intended product into the corresponding benzimidazoline salt of formula III above.

The two salts of formulae I and III can be distinguished as set forth in U. S. Patent No. 4,265,901. The temperature at which the reaction is

conducted is not critical; the reaction goes forward over a wide range of temperatures, such as from 0 to 100° C., but generally room temperature or a slight elevation over room temperature is entirely adequate. If the corresponding benzimidazoline salt of formula III is obtained it can be converted back to the desired benzimidazole salt of formula I by drying, under reduced pressure or by azeotropic distillation. Otherwise, workup of the reaction mixture to isolate the intended product is carried out in routine manner.

The following examples illustrate the preparation of representative salts of formula I and will enable those skilled in the art to practice the invention.

EXAMPLE 1: 2-(1,1,2,2-TETRAFLUOROETHYL)-4-NITRO-6-(TRIFLUOROMETHYL)BENZIMIDAZOLE, TETRA-n-BUTYLAMMONIUM SALT

2-(1,1,2,2-Tetrafluoroethyl)-4-nitro-6-(trifluoromethyl)benzimidazole (33.1 grams) was dissolved in about 200 ml. of methylene chloride and excess tetra-n-butylammonium hydroxide was added. The resulting reaction mixture was stirred for half an hour, then washed with two 50 ml. portions of water, dried over magnesium sulfate, and evaporated, yielding 50 grams of a viscous oil. IR established that the product was 2-hydroxy-2-(1,1,2,2-tetrafluoroethyl)-4-nitro-6-(trifluoromethyl)benzimidazoline, tetra-n-butylammonium salt. The oil was then held for

2 hours at 60° C. and reduced pressure (about 6 mm.) and subsequently cooled to give a solid which was identified by IR and NMR as the intended product, 2-(1,1,2,2-tetrafluoroethyl)-4-nitro-6-(trifluoromethyl)benzimidazole, tetra-n-butylammonium salt. A portion of the product was dissolved in methylene chloride and diluted with isopropyl ether and allowed to stand. In 48 hours, stout needles deposited and were separated, m.p., 90-91° C. Microanalysis:

Calculated for $C_{26}H_{39}F_7N_4O_2$:
C, 54.54; H, 6.87; N, 9.78.
Found: C, 55.47; H, 7.16; N, 9.46.

EXAMPLE 2: 2-(1,1,2,2-TETRAFLUOROETHYL)-4-NITRO-6-(TRIFLUOROMETHYL)BENZIMIDAZOLE, TETRA-n-HEPTYLAMMONIUM SALT

2-Hydroxy-2-(1,1,2,2-tetrafluoroethyl)-4-nitro-6-(trifluoromethyl)benzimidazoline, sodium salt (37.2 grams) in 100 ml of water was stirred while a slurry of tetra-n-heptylammonium iodide (54 grams) in 150 ml. of hot ethanol was added. An additional 150 ml. of water was added and the reaction mixture stirred for 16 hours. Methylene chloride (about 300 ml.) was then added and the layers permitted to separate. Magnesium sulfate was stirred into the methylene chloride layer and filtered and evaporated at 25° C. to a viscous oil. IR indicated that the oil was 2-hydroxy-2-(1,1,2,2-tetrafluoroethyl)-4-nitro-6-(trifluoromethyl)benzimidazoline, tetra-n-heptylammonium salt. The oil was heated to 60° C. for 4 hours under vacuum, then cooled, yielding a solid, the intended 2-(1,1,2,2-tetrafluoroethyl)-4-nitro-6-(trifluoromethyl)benzimidazole, tetra-n-heptylammonium salt, m.p. 45° C.

EXAMPLE 3: 2-(1,1,2,2-TETRAFLUOROETHYL)-4-NITRO-6-(TRIFLUOROMETHYL)BENZIMIDAZOLINE, n-DECYLAMMONIUM SALT

2-(1,1,2,2-Tetrafluoroethyl)-4-nitro-6-(trifluoromethyl)benzimidazole (33 grams) in 200 ml. of diethyl ether was stirred while n-decylamine (15.8 grams in 50 ml. of diethyl ether) was added in one portion. The reaction mixture was then evaporated to dryness, yielding the intended product as a waxy solid, m.p. 92-93° C.

Other salts of formula I were similarly prepared by any of the preceeding examples and by making any change in solvent, temperature and the like as necessary. All salts are a 1:1 ratio unless otherwise indicated. The identity of the products was confirmed by IR and NMR.

EXAMPLE 4: 2-(1,1,2,2-TETRAFLUOROETHYL)-4-NITRO-6-(TRIFLUOROMETHYL)BENZIMIDAZOLE, TRIBENZYLAMMONIUM SALT, m.p. 94° C.

EXAMPLE 5: 2-(1,1,2,2-TETRAFLUOROETHYL)-4-NITRO-6-(TRIFLUOROMETHYL)BENZIMIDAZOLE, TRI-n-BUTYLAMMONIUM SALT, a viscous oil.

EXAMPLE 6: 2-(1,1,2,2-TETRAFLUOROETHYL)-4-NITRO-6-(TRIFLUOROMETHYL)BENZIMIDAZOLE, TETRA-n-PROPYLAMMONIUM SALT, m.p., 130-131° C. Microanalysis:
Calculated for $C_{22}H_{31}F_7N_4O_2$:
C, 51.16; H, 6.05; N, 10.85.
Found: C, 50.96; H, 5.91; N, 10.96.

EXAMPLE 7: 2-(1,1,2,2-TETRAFLUOROETHYL)-4-NITRO-6-(TRIFLUOROMETHYL)BENZIMIDAZOLE, BENZYLTRIMETHYLAMMONIUM SALT, a viscous oil.

EXAMPLE 8: 2-(1,1,2,2-TETRAFLUOROETHYL)-4-NITRO-6-(TRIFLUOROMETHYL)BENZIMIDAZOLE, PHENETHYLAMMONIUM SALT, a yellow solid.

EXAMPLE 9: 2-(1,1,2,2-TETRAFLUOROETHYL)-4-NITRO-6-(TRIFLUOROMETHYL)BENZIMIDAZOLE, DI-n-HEXYLAMMONIUM SALT, a viscous oil.

EXAMPLE 10: 2-(1,1,2,2-TETRAFLUOROETHYL)-4-NITRO-6-(TRIFLUOROMETHYL)BENZIMIDAZOLE, n-DODECYLAMMONIUM SALT, a viscous oil.

EXAMPLE 11: 2-(1,1,2,2-TETRAFLUOROETHYL)-4-NITRO-6-(TRIFLUOROMETHYL)BENZIMIDAZOLE, TRIETHYLAMMONIUM SALT, a viscous oil.

EXAMPLE 12: 2-(1,1,2,2-TETRAFLUOROETHYL)-4-NITRO-6-(TRIFLUOROMETHYL)BENZIMIDAZOLE, TRI-n-PROPYLAMMONIUM SALT, a yellow powder.

EXAMPLE 13: 2-(1,1,2,2-TETRAFLUOROETHYL)-4-NITRO-6-(TRIFLUOROMETHYL)BENZIMIDAZOLE, PROCAINE SALT, a glassy solid melting at 25° C.

EXAMPLE 14: 2-(1,1,2,2-TETRAFLUOROETHYL)-4-NITRO-6-(TRIFLUOROMETHYL)BENZIMIDAZOLE, 2:1 SALT WITH BENZOTHENE, a viscous oil.

EXAMPLE 15: 2-(1,1,2,2-TETRAFLUOROETHYL)-4-NITRO-6-(TRIFLUOROMETHYL)BENZIMIDAZOLE, LEVAMISOLE SALT, a viscous oil.

The salts of formula I exhibit insecticidal action and can be employed for the control of insects in a wide variety of settings. For example, the salts can be employed for the control of insects which attack plants as well as insects which are pests to animals and humans. The salts are particularly useful for the control of colonial insects, household insects, and those insects which attack domestic animals, such as flies, lice, mites, ticks, and fleas. Colonial insects are generally members of the order Hymenoptera, family Formicidae (ants) and the order Isoptera, family Termitidae (termites). The present salts are especially of interest for the control of fire ants, including Solenopsis geminata, Solenopsis richteri, Solenopsis invicta, and Solenopsis xyloni. For the control of ants, the present salt is formulated in an edible bait which is positioned near the colony to be controlled, or, in the case of infested cropland, the edible bait can be distributed uniformly across the infested land. The amount of a present salt to be employed will vary with such factors as the identity of the ant, the size and number of colonies, the mode of application, and the like. Generally, good results are

obtained by application of from 1 to 100 grams/acre, preferably 1 to 10 grams/acre.

Baits containing a present salt of formula I are prepared in manners conventional for the species to be controlled. For many ant species, including fire ants, the present salt is dissolved in an oil or fat which is then distributed on a carrier to constitute the edible bait.

The salts of formula I have been evaluated as toxicants for the control of imported fire ant (Solenopsis richteri). The evaluation was conducted as follows. The candidate compound of formula I was formulated by dissolving it in soybean oil, with heat if necessary. If not soluble upon heating, acetone was added and later removed by evaporation. The resulting solution was placed on defatted pregelled corn grits as carrier, 30% solution and 70% carrier, to constitute a bait suitable for ingestion by ants. Three baits were prepared for each compound, at concentrations of 0.01, 0.1 and 1.0% in the bait.

Imported fire ants were collected from the field, acclimated to laboratory conditions for a week, and then employed in the test. A group of about 40 ants was exposed to the respective bait for 24 hours, then the ants were supplied with an untreated bait of soybean oil on defatted pregelled corn grits carrier for the remainder of the two-week test period. Throughout, the ants were maintained in an environment of controlled temperature and humidity. Dead ants were

removed and counted daily; cumulative percent kill and control were calculated at days 1, 4, 7 and 14 of the test period. Results were as reported in the following table. Each line represents a single test, with individual figures being an average of three replicates. Multiple tests were conducted with the compounds.

Table I

| Compound Tested | Formulation Method | Day 1 % Kill in Treated | Day 1 % Kill in Control | Day 4 % Kill in Treated | Day 4 % Kill in Control | Day 7 % Kill in Treated | Day 7 % Kill in Control | Day 14 % Kill in Treated | Day 14 %Kill in Control |
|---|---|---|---|---|---|---|---|---|---|
| Example 3 | | | | | | | | | |
| 1.00 | 1 | 41 | 0 | 72 | 0 | 81 | 0 | 98 | 53 |
| | | 90 | 0 | 97 | 7 | 99 | 15 | 100 | 27 |
| 0.10 | 1 | 30 | 0 | 70 | 11 | 79 | 13 | 96 | 49 |
| | | 93 | 0 | 100 | 6 | | | | |
| 0.01 | 1 | 3 | 0 | 42 | 7 | 44 | 26 | 88 | 45 |
| | | 6 | 2 | 90 | 21 | 99 | 26 | 100 | 29 |
| Example 4 | | | | | | | | | |
| 1.00 | 1 | 46 | 0 | 84 | 8 | 96 | 11 | 100 | 35 |
| | | 81 | 0 | 97 | 3 | 97 | 3 | 98 | 6 |
| 0.10 | 1 | 60 | 0 | 87 | 0 | 92 | 0 | 100 | 3 |
| | | 96 | 3 | 99 | 12 | 100 | 21 | | |
| 0.01 | 1 | 11 | 0 | 45 | 7 | 66 | 17 | 90 | 57 |
| | | 10 | 5 | 87 | 10 | 97 | 15 | 100 | 18 |

Table I (Continued)

| Compound Tested | Formulation Method | Day 1 % Kill in Treated | Day 1 % Kill in Control | Day 4 % Kill in Treated | Day 4 % Kill in Control | Day 7 % Kill in Treated | Day 7 % Kill in Control | Day 14 % Kill in Treated | Day 14 %Kill in Control |
|---|---|---|---|---|---|---|---|---|---|
| Example 5 | | | | | | | | | |
| 1.00 | 2 | 87 | 0 | 93 | 3 | 96 | 3 | 97 | 67 |
| | | 48 | 0 | 88 | 28 | 100 | 51 | | |
| | | 100 | 0 | | | | | | |
| 0.10 | 2 | 83 | 0 | 97 | 3 | 97 | 9 | 97 | 28 |
| | | 38 | 4 | 76 | 64 | 88 | 81 | 97 | 91 |
| | | 97 | 0 | | | | | | |
| 0.01 | 2 | 2 | 0 | 55 | 3 | 93 | 3 | 99 | 6 |
| | | 17 | 0 | 72 | 56 | 84 | 83 | 95 | 100 |
| | | 17 | 3 | 67 | 3 | 95 | 13 | 98 | 40 |
| Example 6 | | | | | | | | | |
| 1.00 | 3 | 67 | 0 | 89 | 6 | 96 | 25 | 100 | 56 |
| | | 99 | 100 | 0 | | | | | |
| 0.10 | 3 | 49 | 0 | 65 | 5 | 96 | 23 | 99 | 53 |
| | | 95 | 0 | 100 | 12 | | | | |
| 0.01 | 1 | 0 | 0 | 16 | 2 | 42 | 7 | 69 | 10 |
| | | 1 | 0 | 32 | 6 | 52 | 11 | 97 | 17 |

Table I (Continued)

| Compound Tested | Formulation Method | Day 1 % Kill in Treated | Day 1 % Kill in Control | Day 4 % Kill in Treated | Day 4 % Kill in Control | Day 7 % Kill in Treated | Day 7 % Kill in Control | Day 14 % Kill in Treated | Day 14 %Kill in Control |
|---|---|---|---|---|---|---|---|---|---|
| Example 7 | | | | | | | | | |
| 1.00 | 3 | 86 | 4 | 91 | 11 | 93 | 18 | 100 | 36 |
| | | 65 | 2 | 90 | 12 | 97 | 30 | 100 | 63 |
| | | 97 | 3 | 100 | 3 | | | | |
| 0.10 | 3 | 75 | 0 | 98 | 9 | 99 | 15 | 99 | 27 |
| | | 51 | 0 | 74 | 52 | 81 | 85 | 96 | 100 |
| | | 99 | 11 | 100 | 14 | | | | |
| 0.01 | 3 | 7 | 3 | 93 | 8 | 94 | 19 | 96 | 36 |
| | | 33 | 2 | 71 | 15 | 87 | 39 | 98 | 58 |
| | | 26 | 0 | 97 | 6 | 99 | 18 | 100 | 21 |
| Example 8 | | | | | | | | | |
| 1.00 | 3 | 80 | 18 | 96 | 82 | 100 | 100 | | |
| | | 79 | 0 | 91 | 3 | 92 | 3 | 94 | 30 |
| 0.10 | 1 | 81 | 0 | 97 | 0 | 100 | 14 | | |
| | | 96 | 3 | 100 | 3 | | | | |
| 0.01 | 1 | 5 | 2 | 51 | 20 | 64 | 50 | 77 | 93 |
| | | 1 | 0 | 36 | 4 | 75 | 11 | 100 | 11 |

Table I (Continued)

| Compound Tested | Formulation Method | Day 1 % Kill in Treated | Day 1 % Kill in Control | Day 4 % Kill in Treated | Day 4 % Kill in Control | Day 7 % Kill in Treated | Day 7 % Kill in Control | Day 14 % Kill in Treated | Day 14 %Kill in Control |
|---|---|---|---|---|---|---|---|---|---|
| Example 9 | | | | | | | | | |
| 1.00 | 1 | 95 | 3 | 96 | 9 | 97 | 18 | 98 | 61 |
| | | 20 | 0 | 48 | 7 | 71 | 43 | 92 | 86 |
| | | 99 | 3 | | | | | | |
| 0.10 | 1 | 89 | 0 | 100 | 0 | | | | |
| | | 41 | 2 | 76 | 9 | 91 | 26 | 98 | 33 |
| | | 87 | 13 | 100 | 55 | | | | |
| 0.01 | 1 | 2 | 0 | 48 | 17 | 81 | 37 | 98 | 94 |
| | | 16 | 0 | 80 | 42 | 92 | 56 | 98 | 76 |
| | | 17 | 21 | 81 | 75 | 96 | 96 | 100 | 100 |
| Example 10 | | | | | | | | | |
| 1.00 | 1 | 83 | 0 | 91 | 5 | 93 | 19 | 99 | 46 |
| | | 42 | 0 | 58 | 5 | 74 | 18 | 93 | 64 |
| 0.10 | 1 | 95 | 0 | 99 | 6 | 100 | 17 | | |
| | | 34 | 0 | 61 | 12 | 78 | 18 | 97 | 70 |
| 0.01 | 1 | 15 | 0 | 79 | 7 | 92 | 87 | 96 | 100 |
| | | 12 | 0 | 87 | 0 | 100 | 19 | | |
| | | 10 | 3 | 50 | 3 | 71 | 36 | 89 | 86 |
| | | 59 | 0 | 93 | 8 | 97 | 41 | 97 | 77 |

Table I (Continued)

| Compound Tested | Formulation Method | Day 1 % Kill in Treated | Day 1 % Kill in Control | Day 4 % Kill in Treated | Day 4 % Kill in Control | Day 7 % Kill in Treated | Day 7 % Kill in Control | Day 14 % Kill in Treated | Day 14 %Kill in Control |
|---|---|---|---|---|---|---|---|---|---|
| Example 11 | | | | | | | | | |
| 1.00 | 3 | 61 | 0 | 94 | 8 | 100 | 18 | | |
| | | 73 | 0 | 95 | 10 | 97 | 16 | 99 | 26 |
| 0.10 | 3 | 74 | 0 | 95 | 15 | 99 | 38 | 99 | 53 |
| | | 87 | 0 | 96 | 0 | 97 | 4 | 99 | 39 |
| 0.01 | 3 | 27 | 0 | 79 | 5 | 83 | 14 | 90 | 23 |
| | | 11 | 0 | 89 | 28 | 94 | 31 | 99 | 48 |
| Example 12 | | | | | | | | | |
| 1.00 | 2 | 66 | 0 | 94 | 3 | 96 | 3 | 100 | 6 |
| | | 91 | 0 | 97 | 10 | 98 | 10 | 99 | 27 |
| 0.10 | 2 | 78 | 0 | 91 | 5 | 99 | 16 | 100 | 16 |
| | | 93 | 0 | 100 | 0 | | | | |
| 0.01 | 1 | 43 | 0 | 90 | 5 | 97 | 13 | 100 | 23 |
| | | 22 | 0 | 98 | 3 | 99 | 3 | 100 | 16 |

Formulation code =

1 = soluble in soybean oil at room temperature

2 = soluble in heated soybean oil

3 = acetone used to solubilize in soybean oil

As noted above, the salts of formula I are useful as ectoparasiticides for the control of insects which attack domestic animals. For this purpose, the salt can be applied topically or the salt can be administered orally or by injection. The salts are preferably formulated for this use with conventional adjuvants. The amount of salt to be used is not critical and will vary with the identity of the host and the insect parasite, the severity and duration of attack and other factors. In general, good results are achieved at rates of from 1 to about 50 mg./kg. The present salts are advantageous in that when administered to warm blooded animals, they are only slowly taken up by the bloodstream; the present salts therefore provide ectoparasiticidal effect over a period of time. The same delay in being taken up by the bloodstream has the effect of minimizing any toxic action the anion might otherwise exhibit to the host.

Various of the salts of formula I were evaluated for ectoparasiticidal activity. In these tests, the candidate was formulated for administration to cattle, then administered and blood samples thereafter withdrawn daily for a period of time varying from several weeks to several months.

From the blood samples, the serum was isolated and blowfly larvae and adult houseflies were fed on the serum. For the blowfly assay, 10 ml. of serum were placed in a test tube with a wick onto which 50 blowfly larvae were placed. The covered test tube

was incubated at 27° C. for 24 hours, at which time efficacy of the candidate compound was determined as the percent mortality of the blowfly larvae, adjusted by normal mortality in a control, and reported according to the following rating scale:

| Rating | | % Mortality |
|---|---|---|
| 0 | = | none dead |
| 1 | = | 50% dead |
| 2 | = | 51-75% dead |
| 3 | = | 76-90% dead |
| 4 | = | 91-99% dead |
| 5 | = | 100% dead |

For the adult housefly test, 10 ml. of serum were employed to saturate a wick which was placed in a petri dish. Twenty-five chilled houseflies were placed in the dish which was then incubated for 24 hours at 27° C. and 50% relative humidity. After the end of the twenty-four hours, the efficacy of the candidate compound of formula I was determined as the percent mortality of housefly, adjusted as for blowfly by normal mortality in a control and reported according to the same scale as above.

In addition to the blowfly larvae assay and the adult housefly assays, the blood was analyzed for the concentration of the test compound, as the

corresponding 2-hydroxy-2-(1,1,2,2-tetrafluoroethyl)-4-nitro-6-(trifluoromethyl)benzimidazoline anion of formula III.

The results of these evaluations are set forth in the following tables. Each set of data represents a single animal.

Compounds were formulated for injection in either (1) triacetin saturated with water or (2) benzyl alcohol, at a concentration of 200 mg./ml. or 250 mg./ml., respectively; in either instance the indicated amount was as the corresponding 2-hydroxy-2-(1,1,2,2-tetrafluoroethyl)-4-nitro-6-(trifluoromethyl)-benzimidazoline anion. In each evaluation, 5 mg. as the latter anion was supplied per kg. of animal body weight.

TABLE II

TRIACETIN FORMULATION

SUBCUTANEOUS ADMINISTRATION

Compound of Example 3

| Test | | % Efficacy | | | | % Efficacy | | |
|---|---|---|---|---|---|---|---|---|
| Day | PPM | AHF | LBF | SW | PPM | AHF | LBF | SW |
| 0 | | | | | | | | |
| 1 | 3.9 | 100 | 100 | 0 | 2.1 | 24 | 0 | 0 |
| 2 | 4.7 | 100 | 100 | 0 | 4.1 | 100 | 100 | 0 |
| 3 | 4.2 | 100 | 100 | 0 | 5.6 | 100 | 100 | 0 |
| 6 | 5.8 | 100 | 100 | 1 | 8.7 | 100 | 100 | 0 |
| 8 | 6.7 | 100 | 100 | 1 | 8.8 | 100 | 100 | 1 |
| 10 | 7.0 | 100 | 100 | 1 | 7.8 | 100 | 100 | 1 |
| 13 | 5.3 | 100 | 100 | 1 | 6.6 | 100 | 100 | 1 |
| 15 | 4.9 | 100 | 100 | 1 | 6.4 | 100 | 100 | 1 |
| 17 | 3.8 | 96 | 100 | 1 | 5.2 | 89 | 100 | 1 |
| 20 | 2.9 | 41 | 50 | 1 | 4.9 | 100 | 100 | 1 |
| 22 | 2.6 | 36 | 25 | 1 | 4.2 | 83 | 100 | 1 |
| 24 | 2.5 | 48 | 75 | 1 | 3.8 | 83 | 100 | 1 |
| 27 | 2.5 | 20 | 0 | 1 | 4.3 | 75 | 100 | 1 |
| 34 | 2.0 | 23 | 0 | 1 | 2.1 | 19 | 0 | 1 |
| 42 | 1.5 | 20 | 0 | 1 | 1.2 | 15 | 0 | 1 |
| 48 | 1.7 | 24 | 0 | 0 | 1.0 | 19 | 0 | 0 |
| 55 | 1.2 | 17 | 0 | 0 | 0.6 | 18 | 0 | 0 |
| 62 | 0.6 | 16 | 0 | 0 | 0.5 | 26 | 0 | 0 |

Table II (Continued)

Compound of Example 8

| Test | | % Efficacy | | | | % Efficacy | | |
|---|---|---|---|---|---|---|---|---|
| Day | PPM | AHF | LBF | SW | PPM | AHF | LBF | SW |
| 0 | | | | | | | | |
| 1 | 16.2 | 100 | 100 | 0 | 14.2 | 100 | 100 | 0 |
| 2 | 22.8 | 100 | 100 | 0 | 16.7 | 100 | 100 | 0 |
| 3 | 22.3 | 100 | 100 | 0 | 19.8 | 100 | 100 | 0 |
| 6 | 23.4 | 100 | 100 | 0 | 21.3 | 100 | 100 | 0 |
| 8 | 17.9 | 100 | 100 | 1 | 19.6 | 100 | 100 | 1 |
| 10 | 13.6 | 100 | 100 | 1 | 15.3 | 100 | 100 | 1 |
| 13 | 8.2 | 100 | 100 | 1 | 10.8 | 100 | 100 | 1 |
| 15 | 6.2 | 100 | 100 | 1 | 9.5 | 100 | 100 | 1 |
| 17 | 4.4 | 84 | 100 | 1 | 6.8 | 100 | 100 | 1 |
| 20 | 2.0 | 24 | 0 | 1 | 3.2 | 43 | 50 | 1 |
| 22 | 1.2 | 19 | 0 | 1 | 2.3 | 18 | 0 | 1 |
| 24 | 0.7 | 20 | 0 | 1 | 1.9 | 15 | 0 | 1 |
| 27 | 0.4 | 24 | 0 | 1 | 1.7 | 22 | 0 | 1 |
| 34 | 0.1 | 15 | 0 | 1 | 0.3 | 15 | 0 | 1 |
| 42 | NDR | 21 | 0 | 1 | 0.1 | 20 | 0 | 1 |
| 48 | NDR | 25 | 0 | 0 | 0.1 | 24 | 0 | 0 |
| 55 | NDR | 16 | 0 | 0 | NDR | 15 | 0 | 0 |
| 62 | NDR | 26 | 0 | 0 | NDR | 25 | 0 | 0 |

Table II (Continued)

Compound of Example 7

| Test Day | PPM | % Efficacy AHF | LBF | SW | PPM | % Efficacy AHF | LBF | SW |
|---|---|---|---|---|---|---|---|---|
| 0 | | | | | | | | |
| 1 | 18.7 | 100 | 100 | 0 | 16.9 | 100 | 100 | 0 |
| 2 | 18.1 | 100 | 100 | 0 | 19.1 | 100 | 100 | 0 |
| 3 | 18.7 | 100 | 100 | 0 | 17.7 | 100 | 100 | 0 |
| 6 | 12.2 | 100 | 100 | 0 | 13.5 | 100 | 100 | 1 |
| 8 | 12.2 | 100 | 100 | 1 | 13.6 | 100 | 100 | 1 |
| 10 | 10.5 | 100 | 100 | 1 | 10.9 | 100 | 100 | 1 |
| 13 | 7.5 | 100 | 100 | 1 | 8.7 | 100 | 100 | 1 |
| 15 | 5.1 | 100 | 100 | 1 | 6.6 | 100 | 100 | 1 |
| 17 | 3.8 | 88 | 100 | 1 | 3.9 | 84 | 100 | 1 |
| 20 | 2.4 | 27 | 0 | 1 | 2.5 | 36 | 25 | 1 |
| 22 | 2.2 | 16 | 0 | 1 | 1.8 | 19 | 0 | 1 |
| 24 | 1.8 | 20 | 0 | 1 | 1.4 | 23 | 0 | 1 |
| 27 | 1.4 | 21 | 0 | 1 | 0.9 | 25 | 0 | 1 |
| 34 | 0.4 | 18 | 0 | 1 | 0.3 | 25 | 0 | 1 |
| 42 | 0.2 | 19 | 0 | 1 | 0.1 | 15 | 0 | 1 |
| 48 | 0.1 | 15 | 0 | 0 | 0.1 | 29 | 0 | 0 |
| 55 | NDR | 16 | 0 | 0 | NDR | 25 | 0 | 0 |
| 62 | NDR | 16 | 0 | 0 | NDR | 20 | 0 | 0 |

Table II (Continued)

Compound of Example 11

| Test Day | PPM | % Efficacy AHF | LBF | SW | PPM | % Efficacy AHF | LBF | SW |
|---|---|---|---|---|---|---|---|---|
| 0 | | | | | | | | |
| 1 | 27.8 | 100 | 100 | 0 | 34.1 | 100 | 100 | 0 |
| 2 | 31.0 | 100 | 100 | 0 | 31.9 | 100 | 100 | 0 |
| 3 | 28.5 | 100 | 100 | 0 | 29.0 | 100 | 100 | 0 |
| 6 | 17.4 | 100 | 100 | 0 | 17.8 | 100 | 100 | 0 |
| 8 | 15.1 | 100 | 100 | 1 | 14.5 | 100 | 100 | 1 |
| 10 | 11.5 | 100 | 100 | 1 | 10.8 | 100 | 100 | 1 |
| 13 | 6.6 | 100 | 100 | 1 | 6.5 | 100 | 100 | 1 |
| 15 | 5.1 | 100 | 100 | 1 | 4.2 | 100 | 100 | 1 |
| 17 | 3.8 | 96 | 100 | 1 | 3.0 | 84 | 100 | 1 |
| 20 | 2.3 | 29 | 25 | 1 | 1.2 | 21 | 0 | 1 |
| 22 | 1.6 | 15 | 0 | 1 | 0.7 | 22 | 0 | 1 |
| 24 | 1.0 | 16 | 0 | 1 | 0.4 | 15 | 0 | 1 |
| 27 | 0.5 | 24 | 0 | 1 | 0.2 | 20 | 0 | 1 |
| 34 | 0.2 | 17 | 0 | 1 | 0.1 | 21 | 0 | 1 |
| 42 | NDR | 20 | 0 | 1 | NDR | 25 | 0 | 1 |
| 48 | NDR | 21 | 0 | 0 | NDR | 24 | 0 | 0 |
| 55 | NDR | 21 | 0 | 0 | NDR | 20 | 0 | 0 |

Table II (Continued)

Compound of Example 10

| Test Day | PPM | % Efficacy AHF | LBF | SW | PPM | % Efficacy AHF | LBF | SW |
|---|---|---|---|---|---|---|---|---|
| 0 | | | | | | | | |
| 1 | 3.3 | 100 | 100 | 0 | 2.0 | 20 | 0 | 0 |
| 2 | 4.8 | 100 | 100 | 0 | 2.9 | 100 | 100 | 0 |
| 3 | 5.9 | 100 | 100 | 0 | 3.3 | 100 | 100 | 0 |
| 6 | 5.9 | 100 | 100 | 0 | 4.7 | 100 | 100 | 1 |
| 8 | 5.7 | 100 | 100 | 1 | 5.3 | 100 | 100 | 1 |
| 10 | 6.3 | 100 | 100 | 1 | 5.8 | 100 | 100 | 1 |
| 13 | 4.9 | 100 | 100 | 1 | 4.1 | 100 | 100 | 1 |
| 15 | 4.4 | 100 | 100 | 1 | 3.2 | 100 | 100 | 1 |
| 17 | 4.2 | 96 | 100 | 1 | 2.7 | 75 | 100 | 1 |
| 20 | 2.3 | 24 | 25 | 1 | 2.8 | 27 | 0 | 1 |
| 22 | 2.3 | 35 | 25 | 1 | 2.0 | 16 | 0 | 1 |
| 24 | 1.9 | 50 | 75 | 1 | 2.1 | 20 | 0 | 1 |
| 27 | 2.3 | 32 | 50 | 1 | 1.7 | 24 | 0 | 1 |
| 34 | 1.0 | 20 | 0 | 1 | 1.2 | 19 | 0 | 1 |
| 42 | 1.0 | 25 | 0 | 1 | 1.0 | 16 | 0 | 1 |
| 48 | 0.9 | 15 | 0 | 0 | 0.7 | 24 | 0 | 0 |
| 55 | 0.8 | 23 | 0 | 0 | 0.7 | 27 | 0 | 0 |
| 62 | 0.6 | 23 | 0 | 0 | 0.6 | 16 | 0 | 0 |
| 69 | 0.7 | | | | 0.8 | | | |

NDR = no detectable residue ( 0.05 ppm)

SW = swelling score, on scale of 0-4, with 0 = no swelling and 4 = swelling approximately the size of a baseball

TABLE III

FORMULATION IN BENZYL ALCOHOL

INTRAMUSCULAR ADMINISTRATION

Compound of Example 2

| Test | | % Efficacy | | | | % Efficacy | | |
|---|---|---|---|---|---|---|---|---|
| Day | PPM | AHF | LBF | SW | PPM | AHF | LBF | SW |
| 0 | 1.0 | 20 | 0 | 0 | 0.2 | 20 | 0 | 0 |
| 1 | 0.3 | 16 | 0 | 0 | 0.2 | 19 | 0 | 0 |
| 2 | 0.3 | 19 | 0 | 0 | 0.2 | 17 | 0 | 0 |
| 3 | 0.2 | 19 | 0 | 0 | 0.2 | 17 | 0 | 0 |
| 4 | 0.2 | 19 | 0 | 0 | 0.2 | 20 | 0 | 0 |
| 5 | NDR | 19 | 0 | 0 | NDR | 20 | 0 | 0 |
| 6 | 0.1 | 19 | 0 | 0 | 0.1 | 20 | 0 | 0 |
| 7 | 0.2 | 19 | 0 | 0 | 0.1 | 20 | 0 | 0 |
| 8 | 0.2 | 19 | 0 | 0 | 0.2 | 16 | 0 | 0 |
| 9 | 0.2 | 19 | 0 | 0 | 0.1 | 20 | 0 | 0 |
| 10 | 0.2 | 19 | 0 | 0 | 0.1 | 20 | 0 | 0 |
| 11 | 0.2 | 15 | 0 | 0 | 0.1 | 23 | 0 | 0 |
| 12 | 0.2 | 15 | 0 | 0 | 0.1 | 23 | 0 | 0 |
| 13 | 0.2 | 16 | 0 | 0 | 0.2 | 15 | 0 | 0 |

Table III (Continued)

Compound of Example 5

| Test Day | PPM | % Efficacy AHF | LBF | SW | PPM | % Efficacy AHF | LBF | SW |
|---|---|---|---|---|---|---|---|---|
| 0 | 0.5 | 14 | 0 | 0 | 0.1 | 20 | 0 | 0 |
| 1 | 8.3 | 100 | 100 | 0 | 15.8 | 100 | 100 | 0 |
| 2 | 6.0 | 100 | 100 | 0 | 13.0 | 100 | 100 | 0 |
| 3 | 4.0 | 100 | 100 | 0 | 12.4 | 100 | 100 | 0 |
| 4 | 3.2 | 100 | 100 | 0 | 8.9 | 100 | 100 | 0 |
| 5 | 3.6 | 100 | 100 | 0 | 6.0 | 100 | 100 | 0 |
| 6 | 3.5 | 100 | 100 | 0 | 7.8 | 100 | 100 | 0 |
| 7 | 2.9 | 100 | 100 | 0 | 6.5 | 100 | 100 | 0 |
| 8 | 2.9 | 81 | 100 | 0 | 5.0 | 100 | 100 | 0 |
| 9 | 2.4 | 85 | 100 | 0 | 4.7 | 100 | 100 | 0 |
| 10 | 2.4 | 100 | 100 | 0 | 3.6 | 100 | 100 | 0 |
| 11 | 2.3 | 24 | 50 | 0 | 4.2 | 100 | 100 | 0 |
| 12 | 2.2 | 19 | 0 | 0 | 2.5 | 100 | 100 | 0 |
| 13 | 2.4 | 20 | 0 | 0 | 3.5 | 50 | 75 | 0 |
| 14 | | | | | 2.6 | 48 | 75 | 0 |
| 16 | | | | | 2.1 | 16 | 0 | 0 |
| 19 | | | | | 1.6 | 20 | 0 | 0 |

Table III (Continued)

Compound of Example 4

| Test Day | PPM | % Efficacy AHF | LBF | SW | PPM | % Efficacy AHF | LBF | SW |
|---|---|---|---|---|---|---|---|---|
| 0 | NDR | 10 | 0 | 0 | 0.1 | 20 | 0 | 0 |
| 1 | 23.7 | 100 | 100 | 0 | 23.7 | 100 | 100 | 0 |
| 2 | 12.0 | 100 | 100 | 0 | 22.3 | 100 | 100 | 0 |
| 3 | 7.9 | 100 | 100 | 0 | 20.6 | 100 | 100 | 0 |
| 4 | 4.7 | 100 | 100 | 0 | 17.8 | 100 | 100 | 0 |
| 5 | 3.2 | 100 | 100 | 0 | 18.0 | 100 | 100 | 0 |
| 6 | 3.2 | 100 | 100 | 0 | 12.1 | 100 | 100 | 0 |
| 7 | 2.3 | 25 | 0 | 0 | 9.9 | 100 | 100 | 0 |
| 8 | 2.0 | 24 | 0 | 0 | 6.0 | 100 | 100 | 0 |
| 9 | 1.5 | 20 | 0 | 0 | 4.7 | 81 | 100 | 0 |
| 10 | 1.5 | 20 | 0 | 0 | 3.3 | 38 | 50 | 0 |
| 11 | 1.3 | 16 | 0 | 0 | 2.4 | 20 | 0 | 0 |
| 12 | 1.0 | 19 | 0 | 0 | 2.0 | 16 | 0 | 0 |
| 13 | 1.0 | 16 | 0 | 0 | 1.3 | 20 | 0 | 0 |

TABLE IV

FORMULATION IN BENZYL ALCOHOL

SUBCUTANEOUS ADMINISTRATION

Compound of Example 3

| Test | | % Efficacy | | | | %Efficacy | | | | % Efficacy | | |
|---|---|---|---|---|---|---|---|---|---|---|---|---|
| Day | PPM | AHF | LBF | SW | PPM | AHF | LBF | SW | PPM | AHF | LBF | SW |
| 0 | 1.0 | 20 | 0 | 0 | 1.4 | 16 | 0 | 0 | NDR | 19 | 0 | 0 |
| 1 | 15.9 | 100 | 100 | 0 | 4.3 | 100 | 100 | 0 | 6.0 | 100 | 100 | 0 |
| 2 | 6.7 | 100 | 100 | 0 | 9.4 | 100 | 100 | 0 | 17.0 | 100 | 100 | 0 |
| 3 | 6.4 | 100 | 100 | 0 | 9.4 | 100 | 100 | 0 | 14.9 | 100 | 100 | 0 |
| 4 | 5.8 | 100 | 100 | 0 | 9.3 | 100 | 100 | 0 | 11.0 | 100 | 100 | 0 |
| 5 | 6.1 | 100 | 100 | 0 | 9.6 | 100 | 100 | 0 | 8.4 | 100 | 100 | 0 |
| 7 | 6.4 | 100 | 100 | 0 | 10.6 | 100 | 100 | 0 | 8.6 | 100 | 100 | 0 |
| 9 | 5.8 | 100 | 100 | 0 | 9.7 | 100 | 100 | 1 | 8.1 | 100 | 100 | 0 |
| 12 | 5.5 | 100 | 100 | 0 | 7.5 | 100 | 100 | 1 | 5.9 | 100 | 100 | 1 |
| 14 | 5.6 | 100 | 100 | 1 | 7.3 | 100 | 100 | 1 | 4.4 | 100 | 100 | 1 |
| 16 | 5.3 | 100 | 100 | 1 | 5.8 | 100 | 100 | 1 | 4.3 | 100 | 100 | 0 |
| 19 | 4.1 | 100 | 100 | 1 | 4.8 | 100 | 100 | 1 | 2.5 | 14 | 25 | 0 |
| 21 | 4.0 | 100 | 100 | 1 | 3.7 | 100 | 100 | 1 | 1.5 | 20 | 0 | 0 |
| 23 | 3.5 | 100 | 100 | 1 | 3.3 | 83 | 100 | 1 | 1.2 | 19 | 0 | 0 |
| 26 | 3.4 | 100 | 100 | 1 | 1.8 | 20 | 0 | 2 | | | | 1 |
| 28 | 3.1 | 73 | 100 | 1 | 1.6 | 15 | 0 | 2 | | | | |
| 36 | 2.8 | 84 | 100 | 1 | 0.9 | 15 | 0 | 2 | 0.2 | 23 | 0 | 1 |
| 50 | 0.8 | 33 | 0 | 1 | 0.4 | 21 | 0 | 1 | 0.3 | 20 | 0 | 0 |

TABLE IV (Continued)

Compound of Example 9

| Test Day | PPM | % Efficacy AHF | LBF | SW | PPM | %Efficacy AHF | LBF | SW | PPM | % Efficacy AHF | LBF | SW |
|---|---|---|---|---|---|---|---|---|---|---|---|---|
| 0 | 0.8 | 15 | 0 | 0 | NDR | 20 | 0 | | 0.4 | 25 | 0 | 0 |
| 1 | 6.8 | 100 | 100 | 0 | 2.6 | 83 | 100 | 0 | 1.8 | 24 | 0 | 0 |
| 2 | 6.7 | 100 | 100 | 0 | 8.3 | 100 | 100 | 0 | 3.9 | 89 | 100 | 0 |
| 3 | 6.4 | 100 | 100 | 0 | 8.6 | 100 | 100 | 0 | 5.5 | 100 | 100 | 0 |
| 4 | 3.0 | 100 | 100 | 0 | 7.5 | 100 | 100 | 0 | 5.6 | 85 | 100 | 0 |
| 5 | 2.9 | 100 | 100 | 0 | 7.1 | 100 | 100 | 0 | 5.4 | 96 | 100 | 0 |
| 7 | 2.9 | 96 | 100 | 0 | 5.1 | 88 | 100 | 0 | 4.4 | 89 | 100 | 0 |
| 9 | 2.5 | 52 | 75 | 1 | 5.3 | 100 | 100 | 0 | 3.5 | 100 | 100 | 0 |
| 12 | 2.1 | 35 | 50 | 1 | 4.6 | 100 | 100 | 0 | 2.2 | 50 | 75 | 1 |
| 14 | 2.4 | 32 | 25 | 1 | 4.1 | 100 | 100 | 0 | 2.1 | 38 | 50 | 1 |
| 16 | 2.0 | 14 | 0 | 1 | 3.4 | 100 | 100 | 0 | 1.9 | 25 | 0 | 1 |
| 19 | 1.9 | 20 | 0 | 1 | 2.3 | 14 | 0 | 0 | 1.7 | 9 | 0 | 1 |
| 21 | 1.9 | 14 | 0 | 0 | 1.9 | 9 | 0 | 0 | 1.6 | 15 | 0 | 0 |
| 23 | 2.0 | 16 | 0 | 0 | 1.5 | 17 | 0 | 0 | 1.8 | 20 | 0 | 0 |
| 26 | | | | | | | | | | | | |
| 28 | | | | | | | | | | | | |
| 36 | 1.7 | 16 | 0 | 1 | 0.7 | 15 | 0 | 0 | 1.8 | 20 | 0 | 1 |
| 50 | 0.9 | 21 | 0 | 0 | 0.3 | 19 | 0 | 0 | 1.1 | 16 | 0 | 0 |

TABLE V

FORMULATION IN BENZYL ALCOHOL

SUBCUTANEOUS ADMINISTRATION

Compound of Example 2

| Test | | % Efficacy | | | | % Efficacy | | |
|---|---|---|---|---|---|---|---|---|
| Day | PPM | AHF | LBF | SW | PPM | AHF | LBF | SW |
| 0 | NDR | 16 | 0 | 0 | NDR | 16 | 0 | 0 |
| 1 | 0.1 | 14 | 0 | 0 | 0.1 | 20 | 0 | 0 |
| 2 | 0.1 | 14 | 0 | 0 | 0.1 | 20 | 0 | 0 |
| 3 | 0.1 | 16 | 0 | 0 | NDR | 14 | 0 | 0 |
| 4 | 0.1 | 14 | 0 | 0 | 0.1 | 20 | 0 | 0 |
| 5 | 0.1 | 19 | 0 | 0 | 0.1 | 14 | 0 | 0 |
| 6 | NDR | 19 | 0 | 0 | NDR | 20 | 0 | 0 |
| 7 | NDR | 19 | 0 | 0 | NDR | 20 | 0 | 0 |
| 8 | | | | 0 | | | | 0 |
| 10 | | | | | | | | |
| 13 | | | | | | | | |
| 15 | | | | 0 | | | | |
| 17 | | | | | | | | |
| 20 | | | | 0 | | | | |

TABLE V (Continued)

Compound of Example 5

| Test Day | PPM | % Efficacy AHF | LBF | SW | PPM | % Efficacy AHF | LBF | SW |
|---|---|---|---|---|---|---|---|---|
| 0 | NDR | 16 | 0 | 0 | NDR | 16 | 0 | 0 |
| 1 | 12.5 | 100 | 100 | 0 | 13.6 | 100 | 100 | 0 |
| 2 | 10.3 | 100 | 100 | 0 | 9.5 | 100 | 100 | 0 |
| 3 | 8.5 | 100 | 100 | 0 | 10.3 | 100 | 100 | 0 |
| 4 | 7.6 | 100 | 100 | 0 | 12.3 | 100 | 100 | 0 |
| 5 | 7.0 | 100 | 100 | 0 | 11.0 | 100 | 100 | 0 |
| 6 | 7.1 | 100 | 100 | 0 | 10.8 | 100 | 100 | 0 |
| 7 | 5.9 | 100 | 100 | 0 | 10.0 | 100 | 100 | 0 |
| 8 | 5.2 | 81 | 100 | 0 | 7.3 | 84 | 100 | 0 |
| 10 | 3.7 | 100 | 100 | 0 | 7.2 | 100 | 100 | 0 |
| 13 | 1.3 | 16 | 0 | 0 | 4.1 | 77 | 100 | 0 |
| 15 | | | | | 3.0 | 86 | 100 | 0 |
| 17 | | | | | 2.2 | 15 | 50 | 0 |
| 20 | | | | | 1.1 | 20 | 0 | 0 |

TABLE V (Continued)

Compound of Example 4

| Test Day | PPM | % Efficacy AHF | LBF | SW | PPM | % Efficacy AHF | LBF | SW |
|---|---|---|---|---|---|---|---|---|
| 0 | NDR | 16 | 0 | 0 | NDR | 16 | 0 | 0 |
| 1 | 23.7 | 100 | 100 | 0 | 25.9 | 100 | 100 | 0 |
| 2 | 16.5 | 100 | 100 | 0 | 17.7 | 100 | 100 | 0 |
| 3 | 15.0 | 100 | 100 | 0 | 10.6 | 100 | 100 | 0 |
| 4 | 10.3 | 100 | 100 | 0 | 5.1 | 100 | 100 | 0 |
| 5 | 5.2 | 100 | 100 | 0 | 2.4 | 100 | 100 | 0 |
| 6 | 5.7 | 100 | 100 | 0 | 1.0 | 100 | 100 | 0 |
| 7 | 4.0 | 100 | 100 | 0 | 0.4 | 20 | 0 | 0 |
| 8 | 3.1 | 79 | 100 | 0 | | | | 0 |
| 10 | 2.0 | 20 | 0 | 0 | | | | 0 |
| 13 | 0.6 | 16 | 0 | 0 | | | | 0 |
| 15 | | | | 0 | | | | 0 |

TABLE V (Continued)

Compound of Example 6

| Test Day | PPM | % Efficacy AHF | LBF | SW | PPM | % Efficacy AHF | LBF | SW |
|---|---|---|---|---|---|---|---|---|
| 0 | 1.0 | | | | 0.6 | | | |
| 1 | 12.6 | 100 | 100 | 0 | 10.7 | 100 | 100 | 0 |
| 2 | 14.9 | 100 | 100 | 0 | 13.0 | 100 | 100 | 0 |
| 3 | 13.3 | 100 | 100 | 0 | 13.0 | 100 | 100 | 0 |
| 4 | 11.8 | 100 | 100 | 0 | 10.9 | 100 | 100 | 0 |
| 5 | 12.4 | 100 | 100 | 0 | 10.7 | 100 | 100 | 0 |
| 6 | 9.8 | 100 | 100 | 0 | 7.7 | 100 | 100 | 0 |
| 7 | | 100 | 100 | 0 | | 100 | 100 | 0 |
| 8 | | 100 | 100 | 0 | | 100 | 100 | 0 |

TABLE VI

FORMULATION IN TRIACETIN

SUBCUTANEOUS ADMINISTRATION

Compound of Example 8

| Test | | % Efficacy | | | | % Efficacy | | | | % Efficacy | | | | % Efficacy | | |
|---|---|---|---|---|---|---|---|---|---|---|---|---|---|---|---|---|
| Day | PPM | AHF | LBF | SW | PPM | AHF | LBF | SW | PPM | AHF | LBF | SW | PPM | AHF | LBF | SW |
| 0 | | | | | | | | | | | | | | | | |
| 1 | 5.3 | 100 | 100 | 0 | 6.1 | 100 | 100 | 0 | 20.5 | 100 | 100 | 0 | 19.1 | 100 | 100 | 0 |
| 2 | 6.9 | 100 | 100 | 0 | 6.2 | 100 | 100 | | 15.4 | 100 | 100 | | 16.3 | 100 | 100 | 0 |
| 3 | 6.6 | 100 | 100 | 0 | 5.7 | 100 | 100 | | 11.1 | 100 | 100 | | 10.5 | 100 | 100 | 0 |
| 4 | 5.6 | 100 | 100 | 0 | 4.4 | 100 | 100 | 0 | 8.6 | 100 | 100 | 0 | 7.8 | 100 | 100 | 0 |
| 6 | 4.7 | 100 | 100 | 1 | 3.5 | 84 | 100 | 1 | 4.5 | 100 | 100 | 1 | 3.5 | 81 | 100 | 1 |
| 8 | 3.9 | 76 | 100 | 1 | 1.0 | 20 | 0 | 1 | 1.4 | 15 | 0 | 1 | 1.9 | 20 | 0 | 1 |
| 11 | 2.1 | 20 | 0 | 1 | 1.1 | 15 | 0 | 1 | 0.6 | 23 | 0 | 1 | 0.7 | 24 | 0 | 1 |
| 13 | 1.9 | | | | 0.8 | | | | 0.3 | | | | 0.5 | | | |

TABLE VI (Continued)

Compound of Example 11

| Test Day | PPM | % Efficacy AHF | % Efficacy LBF | SW | PPM | % Efficacy AHF | % Efficacy LBF | SW | PPM | % Efficacy AHF | % Efficacy LBF | SW | PPM | % Efficacy AHF | % Efficacy LBF | SW |
|---|---|---|---|---|---|---|---|---|---|---|---|---|---|---|---|---|
| 0 | | | | | | | | | | | | | | | | |
| 1 | 9.4 | 100 | 100 | 0 | 11.6 | 100 | 100 | 0 | 18.3 | 100 | 100 | 0 | 17.8 | 100 | 100 | 0 |
| 2 | 8.0 | 100 | 100 | 0 | 11.8 | 100 | 100 | 0 | 12.1 | 100 | 100 | 0 | 9.7 | 100 | 100 | 0 |
| 3 | 7.2 | 100 | 100 | 0 | 7.8 | 100 | 100 | 0 | 8.0 | 100 | 100 | 0 | 4.8 | 100 | 100 | 0 |
| 4 | 4.6 | 100 | 100 | 0 | 5.2 | 100 | 100 | 0 | 6.4 | 100 | 100 | 0 | 3.0 | 79 | 100 | 0 |
| 6 | 2.2 | 38 | 50 | 1 | 2.2 | 50 | 75 | 1 | 2.5 | 48 | 75 | 1 | 0.9 | 24 | 0 | 1 |
| 8 | 1.3 | 25 | 0 | 1 | 1.1 | 20 | 0 | 1 | 1.1 | 15 | 0 | 1 | 0.6 | 19 | 0 | 1 |
| 11 | 0.8 | 18 | 0 | 1 | 0.4 | 20 | 0 | 1 | 0.4 | 21 | 0 | 1 | 0.1 | 25 | 0 | 1 |
| 13 | 1.0 | | | | 0.4 | | | | 0.3 | | | | 0.0 | | | |

## TABLE VII

### FORMULATION IN TRIACETIN

### SUBCUTANEOUS ADMINISTRATION

### Compound of Example 13

| Test Day | PPM | % Efficacy AHF | LBF | SW | PPM | % Efficacy AHF | LBF | SW | PPM | % Efficacy AHF | LBF | SW | PPM | % Efficacy AHF | LBF | SW | PPM | % Efficacy AHF | LBF | SW |
|---|---|---|---|---|---|---|---|---|---|---|---|---|---|---|---|---|---|---|---|---|
| 0 | NDR | | | | NDR | | | | NDR | | | | NDR | | | | NDR | | | |
| 1 | | 100 | 100 | | | 100 | 100 | | | 100 | 100 | | | 100 | 100 | | | 100 | 100 | |
| | | | | | | | | | | | | | | | | | | Temp:105.6 | | |
| 2 | | 100 | 100 | | | 100 | 100 | | | 100 | 100 | | | 100 | 100 | | | 100 | 100 | |
| | | | | | | Temp:104.5 | | | | | | | | | | | | Temp:105.5 | | |
| 3 | | 100 | 100 | | | 100 | 100 | | | 100 | 100 | | | 100 | 100 | | | 100 | 100 | |
| | | | | | | Temp:103.2 | | | | | | | | | | | | Temp:104.8 | | |
| 4 | 16.1 | 100 | 100 | | 7.1 | 100 | 100 | | 8.7 | 100 | 100 | | 21.0 | 100 | 100 | | 9.0 | 100 | 100 | |
| | | | | | | | | | | | | | | | | | | Temp:104.5 | | |
| 5 | | 100 | 100 | | | 100 | 100 | | | 100 | 100 | | | 100 | 100 | | | 100 | 100 | |
| 6 | | 100 | 100 | | | 100 | 100 | | | 100 | 100 | | | 100 | 100 | | | | | |
| 7 | | 100 | 100 | | | 100 | 100 | | | 100 | 100 | | | 100 | 100 | | | 100 | 100 | |
| 8 | | 75 | 100 | | | 84 | 100 | | | 50 | 75 | | | 76 | 100 | | | 52 | 75 | |
| 11 | | 24 | 0 | | | 25 | 0 | | | 30 | 0 | | | 89 | 100 | | | 29 | 0 | |
| 13 | | 16 | 0 | | | 15 | 0 | | | 14 | 0 | | | 20 | 0 | | | 19 | 0 | |
| 14 | | 24 | 0 | | | 19 | 0 | | | 16 | 0 | | | 18 | 0 | | | 27 | 0 | |
| 17 | | 16 | 0 | | | 15 | 0 | | | 25 | 0 | | | 20 | 0 | | | 23 | 0 | |
| 20 | | 20 | 0 | | | 10 | 0 | | | 23 | 0 | | | 27 | 0 | | | 24 | 0 | |
| 22 | | 24 | 0 | | | 19 | 0 | | | 29 | 0 | | | 30 | 0 | | | 35 | 0 | |
| 26 | | 28 | 0 | | | 28 | 0 | | | 29 | 0 | | | 21 | 0 | | | 28 | 0 | |
| 40 | | 24 | 0 | 0 | | 15 | 0 | 0 | | 30 | 0 | 0 | | 19 | 0 | 0 | | 19 | 0 | 0 |
| 49 | | 24 | 0 | 0 | | 18 | 0 | 0 | | 19 | 0 | 0 | | 16 | 0 | 0 | | 21 | 0 | 0 |

TABLE VII (Continued)

Compound of Example 15

| Test Day | PPM | % Efficacy AHF | LBF | SW | PPM | % Efficacy AHF | LBF | SW | PPM | % Efficacy AHF | LBF | SW | PPM | % Efficacy AHF | LBF | SW | PPM | % Efficacy AHF | LBF | SW |
|---|---|---|---|---|---|---|---|---|---|---|---|---|---|---|---|---|---|---|---|---|
| 0 | NDR | | | | NDR | | | | NDR | | | | NDR | | | | NDR | | | |
| 1 | | 100 | 100 | | | 100 | 100 | | | 100 | 100 | | | 100 | 100 | | | 100 | 100 | |
| 2 | | 100 | 100 | | | 100 | 100 | | | 100 | 100 | | | 100 | 100 | | | 100 | 100 | |
| | | | | | | | | | | Temp:105.2 | | | | 100 | 100 | | | 100 | 100 | |
| 3 | | 100 | 100 | | | 100 | 100 | | | 100 | 100 | | | 100 | 100 | | | 100 | 100 | |
| | | | | | | | | | | Temp:103.9 | | | | 100 | 100 | | | 100 | 100 | |
| 4 | 12.8 | 100 | 100 | | 17.8 | 100 | 100 | | 7.9 | 100 | 100 | | 7.2 | 100 | 100 | | 12.1 | 100 | 100 | |
| 5 | | 100 | 100 | | | 100 | 100 | | | 100 | 100 | | | 100 | 100 | | | 100 | 100 | |
| 6 | | 100 | 100 | | | 100 | 100 | | | 100 | 100 | | | 100 | 100 | | | 100 | 100 | |
| 7 | | 100 | 100 | | | 100 | 100 | | | 100 | 100 | | | 100 | 100 | | | 100 | 100 | |
| 8 | | 85 | 100 | | | 88 | 100 | | | 89 | 100 | | | 20 | 0 | | | 89 | 100 | |
| 11 | | 84 | 100 | | | 83 | 100 | | | 32 | 0 | | | 23 | 0 | | | 84 | 100 | |
| 13 | | 43 | 25 | | | 47 | 50 | | | 24 | 0 | | | 25 | 0 | | | 29 | 0 | |
| 14 | | 23 | 0 | | | 20 | 0 | | | 15 | 0 | | | 20 | 0 | | | 23 | 0 | |
| 17 | | 27 | 0 | | | 15 | 0 | | | 26 | 0 | | | 20 | 0 | | | 15 | 0 | |
| 20 | | 18 | 0 | | | 15 | 0 | | | 24 | 0 | | | 19 | 0 | | | 18 | 0 | |
| 22 | | 16 | 0 | | | 24 | 0 | | | 19 | 0 | | | 29 | 0 | | | 32 | 0 | |
| 26 | | 25 | 0 | | | 24 | 0 | | | 15 | 0 | | | 19 | 0 | | | 28 | 0 | |
| 40 | | 24 | 0 | 0 | | 23 | 0 | 0 | | 16 | 0 | 0 | | 16 | 0 | 0 | | 19 | 0 | 0 |
| 49 | | 16 | 0 | 0 | | 15 | 0 | 0 | | 20 | 0 | 0 | | 18 | 0 | 0 | | 16 | 0 | 0 |

TABLE VII (Continued)

Compound of Example 12

| Test Day | PPM | % Efficacy AHF | LBF | SW | PPM | % Efficacy AHF | LBF | SW | PPM | % Efficacy AHF | LBF | SW | PPM | % Efficacy AHF | LBF | SW | PPM | % Efficacy AHF | LBF | SW |
|---|---|---|---|---|---|---|---|---|---|---|---|---|---|---|---|---|---|---|---|---|
| 0 | NDR | | | | NDR | | | | NDR | | | | NDR | | | | NDR | | | |
| 1 | | 100 | 100 | | | 100 | 100 | | | 100 | 100 | | | 100 | 100 | | | 100 | 100 | |
| 2 | | 100 | 100 | | | 100 | 100 | | | 100 | 100 | | | 100 | 100 | | | 100 | 100 | |
| 3 | | 100 | 100 | | | 100 | 100 | | 15.0 | 100 | 100 | | 18.7 | 100 | 100 | | 22.0 | 100 | 100 | |
| 4 | | 100 | 100 | | | 100 | 100 | | | 100 | 100 | | | 100 | 100 | | | 100 | 100 | |
| 5 | | 100 | 100 | | | 100 | 100 | | | 100 | 100 | | | 100 | 100 | | | 100 | 100 | |
| 6 | | 100 | 100 | | | 100 | 100 | | | 100 | 100 | | | 100 | 100 | | | 100 | 100 | |
| 7 | | 100 | 100 | | | 100 | 100 | | | 100 | 100 | | | 100 | 100 | | | 100 | 100 | |
| 8 | | 85 | 100 | | | 89 | 100 | | | 89 | 100 | | | 83 | 100 | | | 82 | 100 | |
| 11 | | 81 | 100 | | | 82 | 100 | | | 24 | 0 | | | 19 | 0 | | | 84 | 100 | |
| 13 | | 84 | 100 | | | 41 | 25 | | | 20 | 0 | | | 19 | 0 | | | 33 | 25 | |
| 14 | | 75 | 100 | | | 23 | 0 | 1 | | 15 | 0 | 1 | | 23 | 0 | 1 | | 28 | 0 | |
| 17 | | 35 | 25 | 1 | | 24 | 0 | 1 | | 27 | 0 | 0 | | 19 | 0 | 1 | | 23 | 0 | |
| 20 | | 19 | 0 | 1 | | 16 | 0 | 1 | | 21 | 0 | 0 | | 16 | 0 | 1 | | 16 | 0 | |
| 22 | | 20 | 0 | 1 | | 24 | 0 | 0 | | 19 | 0 | 1 | | 22 | 0 | 1 | | 24 | 0 | |
| 26 | | 29 | 0 | 1 | | 29 | 0 | 1 | | 18 | 0 | 1 | | 30 | 0 | 0 | | 15 | 0 | |
| 40 | | 16 | 0 | 1 | | 28 | 0 | 0 | | 16 | 0 | 0 | | 15 | 0 | 0 | | 16 | 0 | 0 |
| 49 | | 19 | 0 | 0 | | 25 | 0 | 0 | | 20 | 0 | 0 | | 16 | 0 | 0 | | 21 | 0 | 0 |

TABLE VII (Continued)

Compound of Example 14

| Test Day | PPM | % Efficacy AHF | % Efficacy LBF | SW | PPM | % Efficacy AHF | % Efficacy LBF | SW | PPM | % Efficacy AHF | % Efficacy LBF | SW | PPM | % Efficacy AHF | % Efficacy LBF | SW | PPM | % Efficacy AHF | % Efficacy LBF | SW |
|---|---|---|---|---|---|---|---|---|---|---|---|---|---|---|---|---|---|---|---|---|
| 0 | NDR | | | | NDR | | | | NDR | | | | NDR | | | | NDR | | | |
| 1 | | 100 | 100 | | | 100 | 100 | | | 100 | 100 | | | 100 | 100 | | | 100 | 100 | |
| 2 | | 100<br>Temp:106.6 | 100 | | | 100 | 100 | | | 100 | 100 | | | 100 | 100 | | | 100 | 100 | |
| 3 | 19.3 | 100<br>Temp:105.3 | 100 | | 13.1 | 100 | 100 | | 13.8 | 100 | 100 | | 16.6 | 100 | 100 | | 20.2 | 100 | 100 | |
| 4 | | 100<br>Temp:107.3 | 100 | | | 100 | 100 | | | 100 | 100 | | | 100 | 100 | | | 100<br>(Died PM) | 100 | |
| 5 | | (Died PM) | | | | 100 | 100 | | | 100 | 100 | | | 100 | 100 | | | | | |
| 6 | | | | | | 100 | 100 | | | 100 | 100 | | | 100 | 100 | | | | | |
| 7 | | | | | | 100 | 100 | | | 100 | 100 | | | 100 | 100 | | | | | |
| 8 | | | | | | 84 | 100 | | | 84 | 100 | | | 88 | 100 | | | | | |
| 11 | | | | | | 80 | 100 | | | 85 | 100 | | | 90 | 10 | | | | | |
| 13 | | | | | | 15 | 0 | | | 32 | 25 | | | 83 | 100 | | | | | |
| 14 | | | | | | 23 | 0 | | | 20 | 0 | | | 76 | 100 | | | | | |
| 17 | | | | | | 20 | 0 | | | 15 | 0 | | | 21 | 0 | | | | | |
| 20 | | | | | | 28 | 0 | | | 28 | 0 | | | 16 | 0 | | | | | |
| 22 | | | | | | 28 | 0 | | | 15 | 0 | | | 25 | 0 | | | | | |
| 26 | | | | | | 28 | 0 | | | 25 | 0 | | | 30 | 0 | | | | | |
| 40 | | | | | | 23 | 0 | 0 | | 19 | 0 | 0 | | 16 | 0 | 0 | | | | |
| 49 | | | | | | 18 | 0 | 0 | | 20 | 0 | 0 | | 15 | 0 | 0 | | | | |

TABLE VIII

FORMULATION IN TRIACETIN

SUBCUTANEOUS ADMINISTRATION

(Blood levels not determined)

Compound of Example 11

| Test | % Efficacy | | | % Efficacy | | | % Efficacy | | | % Efficacy | | | % Efficacy | | |
|---|---|---|---|---|---|---|---|---|---|---|---|---|---|---|---|
| Day | AHF | LBF | SW | AHF | LBF | SW | AHF | LBF | SW | AHF | LBF | SW | AHF | LBF | SW |
| 0 | 11 | 0 | 0 | 14 | 0 | 0 | 11 | 0 | 0 | 5 | 0 | 0 | 15 | 0 | 0 |
| 1 | 89 | 100 | 0 | 84 | 100 | 0 | 95 | 100 | 0 | 81 | 100 | 0 | 100 | 100 | 0 |
| 2 | 89 | 100 | 0 | 90 | 100 | 0 | 90 | 100 | 0 | 95 | 100 | 0 | 86 | 100 | 0 |
| 3 | 90 | 100 | 0 | 89 | 100 | 0 | 86 | 100 | 0 | 95 | 100 | 0 | 100 | 100 | 0 |
| 4 | 95 | 100 | 0 | 89 | 100 | 0 | 84 | 100 | 0 | 89 | 100 | 0 | 90 | 100 | 0 |
| 5 | 95 | 100 | 0 | 80 | 100 | 0 | 84 | 100 | 0 | 89 | 100 | 0 | 84 | 100 | 0 |
| 6 | 95 | 100 | 0 | 88 | 100 | 0 | 85 | 100 | 0 | 86 | 100 | 0 | 89 | 100 | 0 |
| 7 | 100 | 100 | 0 | 100 | 100 | 0 | 100 | 100 | 0 | 89 | 100 | 0 | 100 | 100 | 1 |
| 9 | 24 | 0 | 1 | 89 | 100 | 0 | 88 | 100 | 1 | 100 | 100 | 1 | 95 | 100 | 1 |
| 13 | 24 | 0 | 1 | 89 | 100 | 0 | 23 | 0 | 1 | 63 | 100 | 1 | 84 | 100 | 1 |
| 16 | 16 | 0 | 0 | 83 | 100 | 1 | 20 | 0 | 1 | 24 | 0 | 1 | 75 | 100 | 1 |
| 19 | 16 | 0 | 0 | 85 | 100 | 1 | 24 | 0 | 1 | 20 | 0 | 1 | 15 | 0 | 1 |
| 21 | 20 | 0 | 0 | 38 | 50 | 0 | 20 | 0 | | 19 | 0 | 0 | 20 | 0 | 1 |
| 23 | 16 | 0 | 0 | 38 | 25 | 0 | 20 | 0 | 0 | 19 | 0 | 0 | 16 | 0 | 1 |

**TABLE VIII (Continued)**

**Compound of Example 11**

| Test | % Efficacy | | | % Efficacy | | | % Efficacy | | | % Efficacy | | | % Efficacy | | |
|---|---|---|---|---|---|---|---|---|---|---|---|---|---|---|---|
| Day | AHF | LBF | SW | AHF | LBF | SW | AHF | LBF | SW | AHF | LBF | SW | AHF | LBF | SW |
| 0 | 19 | 0 | 0 | 20 | 0 | 0 | 10 | 0 | 0 | 15 | 0 | 0 | 15 | 0 | 0 |
| 1 | 95 | 100 | 0 | 80 | 100 | 0 | 90 | 100 | 0 | 86 | 100 | 0 | 90 | 100 | 0 |
| 2 | 86 | 100 | 0 | 90 | 100 | 0 | 89 | 100 | 0 | 95 | 100 | 0 | 95 | 100 | 0 |
| 3 | 95 | 100 | 0 | 89 | 100 | 0 | 85 | 100 | 0 | 90 | 100 | 0 | 90 | 100 | 0 |
| 4 | 84 | 100 | 0 | 88 | 100 | 0 | 95 | 100 | 0 | 89 | 100 | 0 | 85 | 100 | 0 |
| 5 | 89 | 100 | 0 | 88 | 100 | 0 | 84 | 100 | 0 | 81 | 100 | 0 | 89 | 100 | 0 |
| 6 | 95 | 100 | 0 | 89 | 100 | 0 | 85 | 100 | 0 | 89 | 100 | 0 | 95 | 100 | 0 |
| 7 | 95 | 100 | 0 | 84 | 100 | 1 | 86 | 100 | 1 | 89 | 100 | 1 | 100 | 100 | 0 |
| 9 | 89 | 100 | 1 | 85 | 100 | 1 | 88 | 100 | 1 | 76 | 100 | 0 | 89 | 100 | 0 |
| 13 | 15 | 0 | 1 | 83 | 100 | 1 | 100 | 100 | 1 | 15 | 0 | 1 | 84 | 100 | 0 |
| 16 | 24 | 0 | 1 | 15 | 0 | 1 | 20 | 0 | 1 | 24 | 0 | 1 | 24 | 0 | 0 |
| 19 | 18 | 0 | 1 | 24 | 0 | 1 | 21 | 0 | 1 | 15 | 0 | 1 | 20 | 0 | 1 |
| 21 | 15 | 0 | 0 | 20 | 0 | 0 | 25 | 0 | 1 | 15 | 0 | 1 | 20 | 0 | 0 |
| 23 | 18 | 0 | 0 | 20 | 0 | 0 | 15 | 0 | 1 | 20 | 0 | 1 | 15 | 0 | 0 |

In a variation of the foregoing test procedures, the compound of Example 12 was evaluated for ectoparasiticidal activity when applied topically. In this procedure, the compound was formulated as follows: 10 grams of compound was dissolved in 10 grams of an oil and 1.5 grams of Cab-O-Sil (a colloidal silica) added. Three grams of the resulting solution was spread onto a 4 cm patch of gauze, backed by tape, and placed in contact with the shaven portion of the animal's ear. This test established the systemic activity of the compound when applied dermally. The results were as set forth in the following table.

TABLE IX

| Test | oil = Capmul 8210 (a mixture of propane-1, 2,3-triol 1-octanoate and 1-decanoate esters, produced by Capital City Products) | | | | | | oil = polyethylene glycol 300 | | | | | |
|---|---|---|---|---|---|---|---|---|---|---|---|---|
| | %Efficacy | | | %Efficacy | | | %Efficacy | | | %Efficacy | | |
| Day | AHF | LBF | SW | AHF | LBF | SW | AHF | LBF | SW | AHF | LBF | SW |
| 0 | 20 | 0 | | 13 | 0 | | | | | | | |
| 1 | 15 | 0 | | 24 | 0 | | 16 | 0 | | 25 | 0 | |
| 2 | 21 | 0 | | 84 | 100 | | 42 | 50 | | 95 | 100 | |
| 5 | 84 | 100 | | 100 | 100 | | 100 | 100 | | 79 | 100 | LT |
| 6 | 38 | 50 | | 95 | 100 | | 100 | 100 | | 75 | 100 | LT |
| 7 | 24 | 0 | LT | 79 | 100 | | 78 | 100 | | 76 | 100 | LT |
| 8 | 20 | 0 | LT | 41 | 50 | | 32 | 50 | | 29 | 25 | LT |
| 9 | 16 | 0 | LT | 55 | 75 | | 13 | 0 | | 19 | 0 | LT |
| 12 | 19 | 0 | LT | 16 | 0 | | 19 | 0 | | 20 | 0 | LT |
| 13 | 15 | 0 | LT | 20 | 0 | | 14 | 0 | | 20 | 0 | LT |
| 14 | 20 | 0 | LT | 15 | 0 | | | | | | | |
| 16 | 14 | 0 | LT | 19 | 0 | | | | | | | |

CLAIMS

1. A benzimidazole salt of the formula

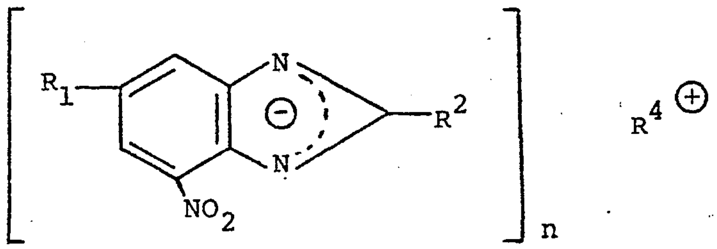

wherein $R^1$ is bromo, chloro, $-CF_3$, $-CF_2Br$, or $-CF_2Cl$; $R^2$ is perfluoroalkyl of $C_1$-$C_3$ or $-CF_2-CF_2R^3$ wherein $R^3$ is hydrogen or $C_1$-$C_6$ alkyl; $R^4$ is a substituted ammonium cation derived from an organic amine; and n is the valence of $R^4$, the said salt having a $K_{OW}$ >150 in the system octanol/water.

2. A compound of Claim 1 wherein $R^1$ is trifluoromethyl and $R^2$ is 1,1,2,2-tetrafluoroethyl.

3. Any one of the following compounds;
2-(1,1,2,2-tetrafluoroethyl)-4-nitro-6-(trifluoromethyl)benzimidazole, tetra-n-propylammonium salt;
2-(1,1,2,2-tetrafluoroethyl)-4-nitro-6-(trifluoromethyl)benzimidazole, tetra-n-heptylammonium salt;
2-(1,1,2,2-tetrafluoroethyl)-4-nitro-6-(trifluoromethyl)benzimidazole, n-decylammonium salt;
2-(1,1,2,2-tetrafluoroethyl)-4-nitro-6-(trifluoromethyl)benzimidazole, tribenzylammonium salt;
2-(1,1,2,2-tetrafluoroethyl)-4-nitro-6-(trifluoromethyl)benzimidazole, tri-n-butylammonium salt;

2-(1,1,2,2-tetrafluoroethyl)-4-nitro-6-(trifluoromethyl)benzimidazole, benzyltrimethylammonium salt; 2-(1,1,2,2-tetrafluoroethyl)-4-nitro-6-(trifluoromethyl)benzimidazole, phenethylammonium salt; 2-(1,1,2,2-tetrafluoroethyl)-4-nitro-6-(trifluoromethyl)benzimidazole, di-n-hexylammonium salt; 2-(1,1,2,2-tetrafluoroethyl)-4-nitro-6-(trifluoromethyl)benzimidazole, n-dodecylammonium salt; 2-(1,1,2,2-tetrafluoroethyl)-4-nitro-6-(trifluoromethyl)benzimidazole, triethylammonium salt; and 2-(1,1,2,2-tetrafluoroethyl)-4-nitro-6-(trifluoromethyl)benzimidazole, tri-n-propylammonium salt.

4. An ectoparasiticidal formulation comprising as an active ingredient a benzimidazole salt of formula I, as claimed in any one of Claims 1 to 3, associated with one or more acceptable carriers or diluents therefore.

5. A process for preparing a benzimidazole salt of the formula

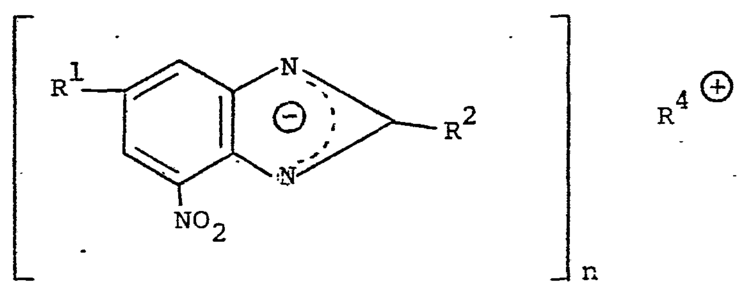

(I)

wherein $R^1$ is bromo, chloro, $-CF_3$, $-CF_2Br$, or $-CF_2Cl$; $R^2$ is perfluoroalkyl of $C_1$-$C_3$ or

$-CF_2-CF_2R^3$ wherein $R^3$ is hydrogen or $C_1-C_6$ alkyl; $R^4$ is a substituted ammonium cation derived from an organic amine; and n is the valence of $R^4$, the said salt having a $K_{OW}$ >150 in the system octanol/water, which comprises reacting a compound of the formula

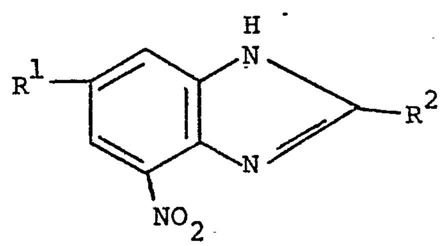

(II)

wherein $R^1$ and $R^2$ are defined as before

(1) with an organic amine, $R^4$, which is defined as before, in the presence of an aprotic solvent; or

(2) with an alkali metal hydroxide or an alkali metal carbonate, followed by reacting the alkali metal salt with an appropriate salt of the $R^4$ organic amine, in the presence of a protic solvent; or

(3) with an organic amine, $R^4$, which is defined as before, or with an appropriate salt of the $R^4$ organic amine, in the presence of a protic solvent;

and step (2) or (3) above is further followed by drying the resulting benzimidazoline salt of the formula

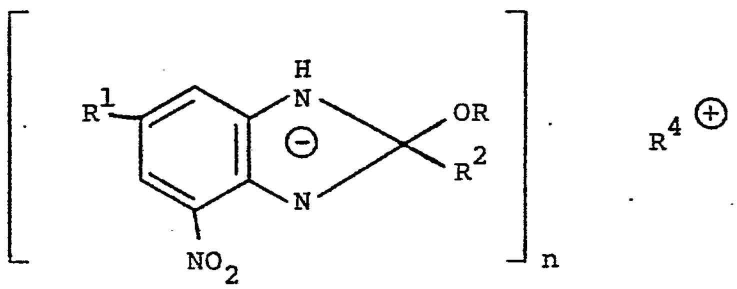

(III)

wherein $R^1$, $R^2$, $R^4$ and n are defined as before, and R is hydrogen or $C_1$-$C_4$ alkyl.

European Patent Office

# EUROPEAN SEARCH REPORT

0113945

Application number

EP 83 30 0259

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int. Cl. 3) |
| A | US-A-3 755 346 (SOPER)<br>* Columns 2,12 *<br>--- | 1-5 | C 07 D 235/10<br>A 01 N 43/52 |
| A | US-A-4 122 184 (SOPER)<br>--- | | |
| D,X | EP-A-0 031 699 (ELI LILLY)<br>* Pages 4-7 *<br>----- | 1-3,5 | |
| | | | TECHNICAL FIELDS SEARCHED (Int. Cl. 3) |
| | | | C 07 D 235/00<br>A 01 N 43/00 |

The present search report has been drawn up for all claims

| Place of search | Date of completion of the search | Examiner |
|---|---|---|
| THE HAGUE | 16-09-1983 | DE BUYSER I.A.F. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82